# EUROPEAN PATENT SPECIFICATION

(11) **EP 1 080 206 B1**
(45) Date of publication and mention of the grant of the patent: **02.04.2003**
(21) Application number: 99923745.6
(22) Date of filing: 21.05.1999
(51) Int. Cl.: C12N 15/62, C12N 15/63, C12N 5/10, A61K 48/00

(54) **GENERATION OF MULTIPLE GENE PRODUCTS FROM CHIMERIC FUSION PROTEINS BY CLEAVAGE WITH ENDOPROTEASES**
HERSTELLUNG EINZELNER GENPRODUKTE EINES CHIMÄREN FUSIONSPROTEINS DURCH SPALTUNG MIT ÜBERALL VORKOMMENDEN ENDOPROTEASEN
GENERATION DE PRODUITS GENIQUES MULTIPLES A PARTIR DE PROTEINES CHIMERES DE FUSION, PAR CLIVAGE A L'AIDE D'ENDOPROTEASES

(30) Priority: 21.05.1998 GB 9810999
(43) Date of publication of application: 07.03.2001
(73) Proprietor: Cancer Research Technology Limited, London WC2A 3PX (GB)
(72) Inventor: Gäken, Johannes Adrianus, London SE5 9NU (GB); Farzaneh, Farzin, London SE5 9NU (GB); Russell, Stephen James, Rochester, MN 55905 (US)
(74) Representative: Cripps, Joanna Elizabeth
(86) International application number: GB9901609
(87) International publication number: WO99060135

(56) References cited:
- EP-A- 0 212 532
- WO-A-91/02065
- WO-A-97/12048
- GROS, L. ET AL.: "Regulated production of mature insulin by non-beta cells" HUMAN GENE THERAPY, vol. 8, no. 18, 10 December 1997 (1997-12-10), pages 2249-2259, XP002116724
- SEIDAH, N.G. AND CHRETIEN, M.: "Eukaryotic protein processing: Endoproteolysis of precursor proteins" CURRENT OPINION IN BIOTECHNOLOGY, vol. 8, no. 5, October 1997 (1997-10), pages 602-607, XP002116725
- GERMINO, J. AND DEEPAK, B.: "Rapid purification of a cloned gene product by genetic fusion and site-specific proteolysis" PROCEEDINGS OF THE NATIONAL ACADEMY OF SCIENCES, vol. 81, August 1984 (1984-08), pages 4692-4696, XP002116726
- HE, X.-S. ET AL.: "Construction of adenoviral and retroviral vectors coexpressing the genes encoding the hepatitis B surface antigen and B7-1 protein" GENE, vol. 175, no. 1-2, 10 October 1996 (1996-10-10), pages 121-125, XP004043303
- NAKAYAMA, K.: "Furin: A mammalian subtilisin/Kex2P-like endoprotease involved in processing of a wide variety of precursor proteins" BIOCHEMICAL JOURNAL, vol. 327, no. pt3, 1 November 1997 (1997-11-01), pages 625-635, XP002116727
- GAEKEN, J. ET AL.: "Irridiated NC adenocarcinoma cells transduced with both B7.1 and Interleukin-2 induce CD4+-mediated rejection of established tumours" HUMAN GENE THERAPY, vol. 8, no. 4, 1 March 1997 (1997-03-01), pages 477-488, XP002116728
- Gaeken, J. et al.:"Fusagen vectors: A novel strategy for the expression of multiple genes from a single cistron" CANCER GENE THERAPY vol.5, no. 6 (Conf. Suppl.), Nov./Dec.1998, page s12-s13 Seventh International Conference on Gene Therapy of Cancer, San Diego, California, USA, 19-21.11.1998 XP002116730
- SHORT, D.K. ET AL.: "Adenovirus-mediated transfer of a modified human proinsulin gene reverses hyperglycemia in diabetic mice" AMERICAN JOURNAL OF PHYSIOLOGY, vol. 275, no. 5 PT 1, November 1998 (1998-11), pages E748-E756, XP002116729

## Description

### Field of the invention

The present invention concerns materials and methods relating to the expression of genes. Particularly, but not exclusively, it relates to the methods for expressing multiple gene products as a single cistron, the therapeutic use of such methods and kits for carrying out the methods.

### Background of the invention

Transfer and expression of multiple exogenous genes is an important requirement in a range of gene therapy protocols, including immune gene therapy of cancer. However, the relative inefficiency of gene transfer procedures, precludes the use of multiple vectors for the transfer and expression of multiple gene products. In principle, expression of several genes in a single vector can be achieved by inclusion in the vector of multiple expression cassettes each with its own independent promoter and/or transcriptional termination sequences. A major disadvantage of this approach is the phenomenon of promoter interference, which is particularly acute in retroviral vectors (Emerman and Temin, 1984 Cell 39, 449-467; Emerman and Temin, 1986 Nucleic Acid Res. 14, 9381-9396; Vile et al., 1994 Gene Ther. 1, 307-316). As an alternative strategy, the recent development of retroviral vectors containing internal ribosome entry sites (IRES) has enabled expression of polycistronic messengers (Morgan et al., 1992 Nucleic Acids. Res. 20, 1293-1299; Zitvogel et al., 1994 Hum. Gene Ther. 5, 1493-1506). However, these vectors are most efficient when they contain only a single IRES, limiting their suitability to expression of only two genes. The presence of multiple IRES elements result in vector instability, probably because of recombination events. In the inventors' experience this is the case even in vectors with two IRES sequences derived from different viral species (e.g. polio virus and encephalomyocarditis virus ECMV) with little nucleotide sequence homology. Therefore, such vectors are unsuitable for the delivery and expression of multiple gene products. Accordingly, the present inventors have realized that there is a need for a new approach for the expression of multiple gene products from a single cistron which encodes multiple products as a single fusion protein which can be cleaved and processed after synthesis into its constituent, biologically active, components.

In eukaryotic cells many gene products (e.g. prohormones, neuropeptide precursors, etc.) are synthesized initially as precursor molecules which are processed post-translationally by proteolytic cleavage (see refs (Barr, 1991 DNA Cell Biol. 10, 319-328; Steiner et al., 1992 J. Biol. Chem. 267, 23435-23438) for reviews). A number of tissue specific endoproteases which are expressed in pancreatic islet, pituitary gland and other neuroendocrine cells have been identified (PC2, PC3, PC4, PACE4) (Smeekens and Steiner, 1990 J. Biol. Chem. 265, 2997-3000; Smeekens et al., 1991 Proc. Natl. Acad. Sci. U.S.A. 88, 340-344; Nakayama et al., 1992 J Biol. Chem. 267, 5897-5900; Kiefer et al., 1991 DNA Cell Biol. 10, 757-769) by their homology to Kex2, the first eukaryotic processing endoprotease identified in yeast (Julius et al., 1984 Cell 37, 1075-1089; Mizuno et al., 1988 Biochem. Biophys Res. Commun. 156, 246-254). In contrast, furin which is an endoprotease localized to the Golgi apparatus (Shapiro et al., 1997 J. Histochem. Cytochem. 45, 3-12 ), is expressed in a wide variety of tissues (Schalken et al., 1987 J. Clin. Invest. 80, 1545-1549), and is highly conserved among eukaryotic species (Barr et al., 1991 Cell 66, 1-3). Furin represents a general cellular endoprotease able to processes a diverse group of precursor proteins secreted via the constitutive pathway. Most prohormones and neuroendocrine precursors are processed at Lys-Arg or Arg-Arg dibasic residues whilst the precursors of proteins secreted via unregulated or constitutive pathways, have a more complex furin cleavage site with the consensus sequence Arg.X.Arg/Lys.Arg (Bresnahan et al., 1990 J. Cell Biol. 111, 2851-2859; Hosaka et al., 1991 J. Biol. Chem. 266, 12127-12130). Direct evidence for the proteolytic activity of furin was obtained by co-transfection experiments with cDNAs for furin and von Willebrand factor precursor (pro-vWF) introduced into COS cells. The presence of furin resulted in the full maturation of pro-vWF at it's natural Arg-Ser-Lys-Arg processing site and showed the importance of Arg at the P4 position (van de Ven et al., 1990 Mol. Biol. Rep. 14, 265-275 ; Wise et al., 1990 Proc. Natl. Acad. Sci. USA 87, 9378-9382). A number of studies have shown that incorporation of furin recognition sites into recombinant proteins result in the efficient secretion of these proteins from transduced cells. These studies include secretion of a peptide encoding the Fc fragment of IgG1, as a result of its fusion to the N-terminal domain of pro-calcitonin (Liu et al., 1993 Proc. Natl. Acad. Sci. USA 90, 8957-8961). Similarly, secretion of mature active human insulin can be achieved in cells which express only the constitutive pathway of secretion as a result of incorporation of furin recognition sites into the recombinant pro-insulin (Groskreutz et al., 1994 J. Biol. Chem. 269, 6241-6245).

The expression of multiple gene products is a requirement in a number of biological applications. A number of the already available techniques allow this objective, but with limitations. These include sequential gene transfer using vectors each of which encode a single or multiple genes as independently regulated expression units. Alternatively the use of IRES sequences placed between different coding sequences allow the expression of polycistronic transcripts in which each coding unit is then translated into the corresponding protein product.

However, each of these procedures have a number of disadvantages. The use of multiple plasmids is time consuming, inefficient and usually dependent on the availability of different selection methods for each of the encoded genes. The incorporation of multiple expression cassettes can result in promoter interference, where the expression from one cassette influences (usually suppresses) the expression of the other expression cassette(s) in the vector (Emerman and Temin, 1984 Cell 39, 449-467; Emerman and Temin, 1986 Nucleic Acid Res. 14, 9381-9396;). The incorporation of a single IRES results in efficient expression of two genes but incorporation of two or more IRES sequences for the expression of more than two genes makes such vectors unstable. Furthermore, non of these methods allow the coordinated expression of the encoded genes, and therefore entirely unsuitable for situations in which equimolar (i.e. 1:1) or indeed other specific molar ratios (e.g. 2:1) of two or more gene products are required. This is a particularly important consideration for gene transfer studies in which the desired biological activity is the product of heterogeneous subunits (e.g. expression of the p40 subunit of IL-12 in molar excess to the p35 subunit results in inhibition of the immune stimulatory activity of IL-12 (Chen et al., 1997 J. Immunol. 159, 351-359; Mattner et al., 1997 Infect. Immun. 65, 4734-4737).

Two previous studies have made use of furin cleavage sites, not for the production of multiple proteins from a single cistron, but for the secretion of proteins that are normally intracellular (Liu et al., 1993 Proc. Natl. Acad. Sci. U.S.A. 90, 8957-8961) or for the production of peptides that are normally secreted, but only by specific cell types (Methot et al., 1997 J. Biol. Chem. 272, 12994-12999). In the first study, as mentioned above, an antibody Fc fragment was fused via a furin cleavage site to the pro-calcitonin signal peptide, resulting in the secretion of the Fc fragment (Liu et al., 1993 Proc. Natl. Acad. Sci. U.S.A. 90, 8957-8961). In the second study angiotensin II was fused via the furin recognition site to a portion of prorenin containing its signal peptide, thus enabling the secretion of angiotensin II by cells which lack the complete modification system needed for the production of the mature peptide (Methot et al., 1997 J. Biol. Chem. 272, 12994-12999).

He et al (Gene 175 (1996) 121-125) used an internal ribosomal entry site of encephalomyocarditis virus between two cooling sequences to form a dicistronic DNA fragment and showed that the two sequences were expressed in the human cell line.

### Summary of the invention

The present inventors have appreciated that endoprotease cleavage sites, for example furin recognition sites, may be utilised to produce multiple gene products from chimeric fusion proteins by endoproteolytic cleavage to generate the constituent proteins, each with its own specific biological activity being either individually, in complex with each other or in association with other cellular components. In addition, the present inventors have realised that this technology allows expression of two or more polypeptides (proteins), these being the constituents of the chimeric fusion protein, in either equimolar ratios, or other ratios as specified by the frequency of reiteration of a given protein or peptide sequence within the fusion protein. Furthermore, the site of endoproteolytic cleavage incorporated in the chimeric protein may be designed to be a target for ubiquitous endoproteases (e.g. furin) and therefore generate the constituent proteins in many different cell types. Alternatively the cleavage site may be designed to be a target for cell specific endoproteases and therefore generate the constituent proteins in specific target cells which express such endoproteases. The latter provides a novel mechanism for tissue specific expression of proteins which may be used as a strategy either in conjunction, or separately from the previously described means of achieving tissue specific expression (i.e. targeted delivery and tissue specific transcription).

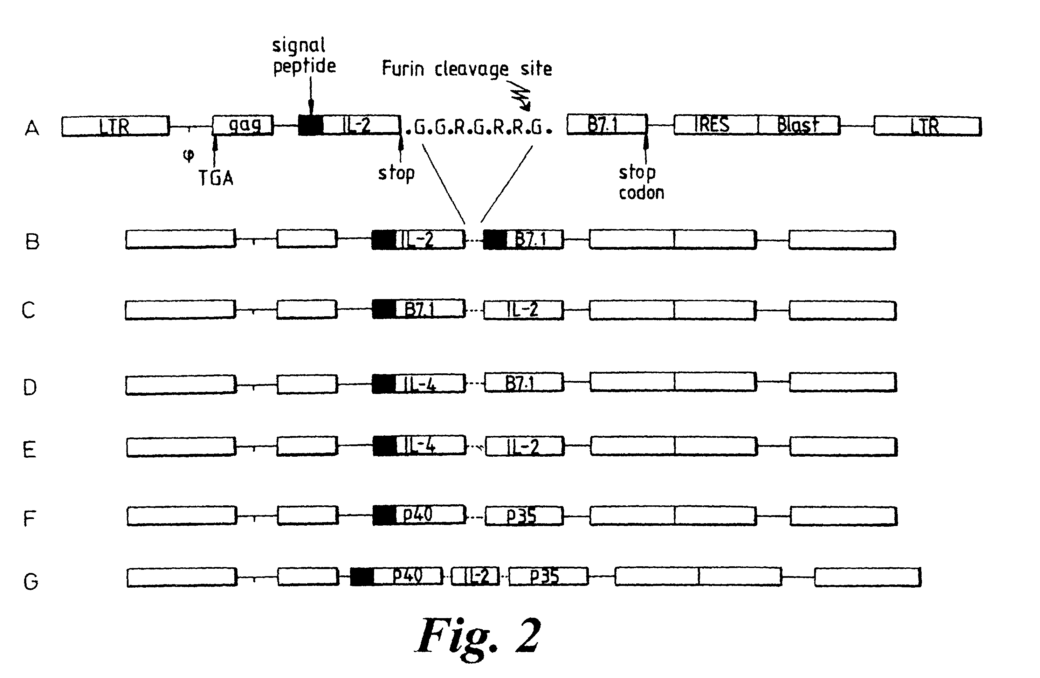

The present inventors have, by way of an example, expressed IL-2 and B7.1, IL-4 and B7.1, IL-4 and IL-2, IL-12 (composed of two subunits, p35 and p40), or IL-12 and IL-2 (composed of three subunits, p35 and p40 of IL-12 plus IL-2) as a fusion protein, separated in each case by a linker encompassing a furin target sequence. In these examples, the furin cleavage site used was Gly.Gly.Arg.Gly.Arg.Arg.Gly for the B7.1/IL2 encoding vectors (Fig. 2 A, B and C), Gly.Gly.Arg.Tyr.Arg.Arg.Gly.Gly for the IL-4 encoding vectors (Fig. 2 D and E), or Gly.Arg.Pro.Lys.Arg.Pro for the IL-12 encoding vectors (Fig. 2 F and G). These sequences encompass a consensus furin cleavage site (in these examples Arg.Gly.Arg.Arg.or Arg.Pro.Lys.Arg) and additional glycine or proline residues to make the cleavage site spatially more accessible. Furin cleavage sites usually conform to the consensus sequence Arg.X.Y.Arg. in which X can be any amino acid, for example Glycine, Lysine, Valine, Tyrosine, Glutamine or Proline, and Y is preferably Lysine or Arginine. The expression of a third gene, blasticidin S deaminase, via an ECMV IRES sequence, allows the isolation of the successfully transduced cells, in each of which the constituents of the fusion gene are expressed as biologically active material in the appropriate subcellular location. The expression of biologically active human IL-2 and IL-4 have been confirmed by CTLL assays and ELISA, and for Il-12 by activation of human lymphocytes and ELISA. The expression of biologically active human B7.1 has been confirmed by FACS analysis using a monoclonal antibody or the natural ligand CTLA4, and by its ability to co-stimulate the production of IL-2 by the Jurkat T-cell line.

Therefore, at its most general, the present invention provides materials and methods for generating multiple gene products from a single cistron encoding multiple products as a single fusion protein which can be cleaved and processed after synthesis into its constituent, biologically active, components.

In a first aspect, the present invention provides a nucleic acid construct for expressing two or more polypeptides in a target cell as a fusion protein said construct comprising a first sequence encoding a first polypeptide and a second sequence encoding a second polypeptide, said first and second sequences being separated by a linker sequence encoding an endoprotease cleavage site.

The endoprotease cleavage site may be any amino acid sequence that is recognised by an endoprotease. Examples of endoproteases include PC2, PC3, PC4, PACE4 and Kex2. Preferably, the endoprotease is furin which recognises a cleavage site having the amino acid sequence Arg.X.Y.Arg where X is any amino acid residue and Y is preferably Lysine or Arginine. It is well within the capabilities of the skilled person to provide alternative cleavage sites which are recognised and/or targeted by other endoproteases which may have desired alternative sub-cellular localisations.

In order for the endoprotease cleavage site to be spatially more accessible to the endoprotease, it is preferable for the linker sequence to further comprise spacer sequence. Such spacer sequence may simply be one or more suitable additional codons (e.g. glycine encoding codons) positioned either side of the nucleotides encoding the endoprotease cleavage site. The cleavage site may be encoded by any DNA sequence specifying an amino acid sequence suitable for endoprotease cleavage, and preferably flanked with a small number (2, 3, 4 etc) of suitable additional amino acids to make the cleavage site more accessible to the endoprotease. Where the cleavage site is a furin cleavage site, it may be encoded by any DNA sequence suitable for furin cleavage (e.g. Arg. X.Y.Arg, as described above). As an example, the linker sequence may be AGG-GGT-CGA-CGC. This cleavage site may be flanked at each end by additional codons encoding glycine residues as spacer sequence, for example, GGC-GGC-AGG-GGT-CGA-CGC-GGC-GGC which encodes Gly. Gly. Arg. Gly. Arg. Arg. Gly. Gly. The linker sequence may also comprise the consensus sequence MGN-NNN (excluding TRR)-AAR-MGN or MGN-NNN (excluding TRR) MGN-MGN, as defined by International Union of Pure and Applied Chemistry (Biochemical Nomenclature and Related Documents, 2nd Edition Portland Press, 1992 pp122-126).

The nucleic acid construct may be DNA, cDNA, RNA, linear or circular, single or double stranded such that the two or more polypeptides are encoded as a monocistronic transcript. Preferably, the construct forms part of a vector which can be used to transfect or infect a host cell. Vectors and host cells comprising such a nucleic acid construct form further aspects of the present invention.

In a second aspect, the present invention provides an in vitro or ex vivo method for expressing two or more polypeptides in a target cell, said method comprising the steps of
a) constructing a nucleic acid construct as defined above; and
b) transfecting said target cell with said nucleic acid construct such that said nucleic acid construct is expressed to produce a fusion protein comprising said first and second polypeptides linked by an endoprotease cleavage site, said cleavage site being cleaved by an endoprotease within the target cell to generate individual biologically active first and second polypeptides.

Preferably, the nucleic acid construct is part of a nucleic acid vector, particular a viral vector, for example a retroviral vector. The retroviral vector may be transfected into a retroviral packaging cell line. These cells may then be used to infect the target cell.

The construct and method of the present invention may be used as research tools for studying the expression and activity of two or more polypeptides in a target cells. This may provide production of multi-subunit proteins or protein complexes which after isolation from the target cells can be used for industrial, diagnostic or therapeutic purposes. Alternatively, the target cells themselves may be used for such purposes, including the generation of cellular vaccines (e.g. tumour cell vaccines) which are transformed with vectors encoding for such chimeric proteins expressing one or more biologically active products (e.g. IL-12, which is composed of two different subunits). The method may also be used for the ectopic expression of multiple proteins in cell which do not normally express these proteins. The method primarily provides the advantageous ability to generate two or more proteins at specified molar ratios. This has particular implications with regard to the expression of proteins that have multiple subunits (e.g. IL-12) that require expression of the independent subunits at equimolar amounts. Indeed, subunits expressed in differing molar quantities can produce an antagonistic effect with regard to protein combination.

In a further application of the technology provided herein, the incorporated endoprotease recognition and/or cleavage site or sites may be for cleavage by endoproteases including tissue specific endoproteases, thus providing a novel mechanism (other than targeted delivery or tissue specific transcription) for tissue specific processing of single proteins, or chimeric proteins composed of multiple components, which may be inactive prior to the endoproteolytic cleavage and be activated as a result of this proteolytic cleavage, thus allowing tissue specific expression of the biological activity or activities encoded by individual constituents of the chimeric protein, or by their concerted action together, or with other cellular components.

Alternatively, the technology according to the present invention may be used for the treatment of diseases where cell types can be targeted for the production of two or more polypeptides. Such treatments may include gene therapy, to treat a patient who is unable to synthesize the active polypeptides or unable to synthesize them at the normal level or in association, thereby providing the effect provided by the wild-type polypeptides i.e. the encoded polypeptides themselves or products of their further modifications. This is described in more detail below.

### Brief Description of the Drawings

Aspects and embodiments of the present invention will now be described by way of example only, with reference to the accompanying figures. Further aspects and embodiments will be. apparent to those skilled in the art.

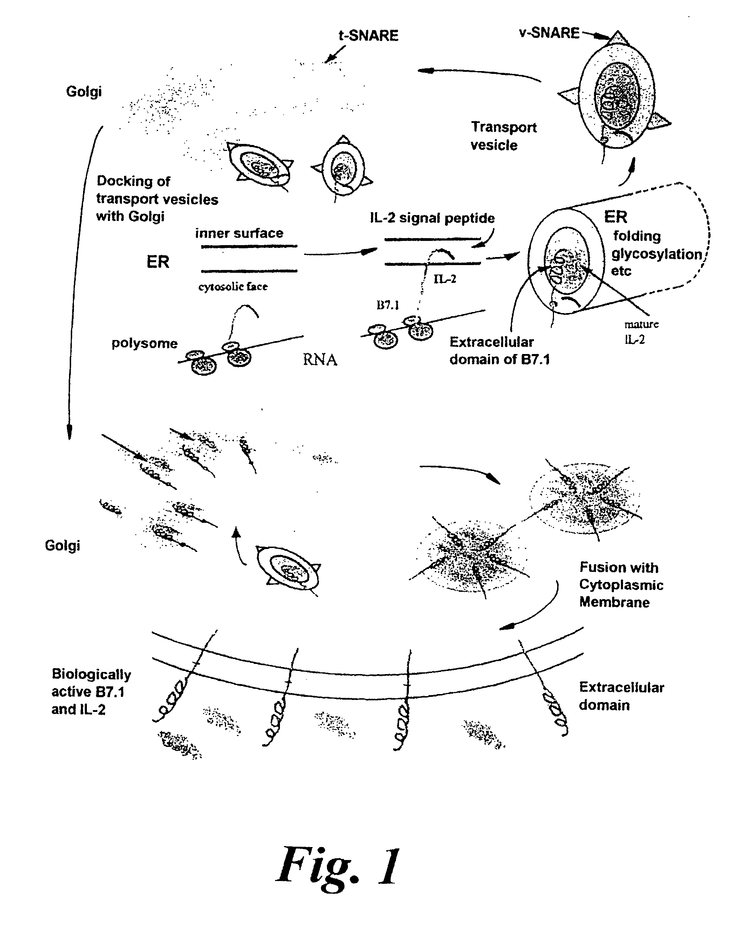

**Figure 1** shows a schematic overview of the constitutive pathway of secretion and the proposed way of action of the furin endoprotease on chimeric proteins.
**Figure 2** shows a schematic representation of the 7 retroviral vectors containing different combinations of IL-2, IL-4, IL-12 p35, IL-12 p40 and B7.1 "fusagene" constructs.
**Figure 3** shows the cell surface expression of B7.1 on AM12 cells infected with pWZLB7/IL2M, pWZLIL4/B7M and pWZLIL2B7M detected by flow cytometry using CTLA4-Ig and FITC-Ig.
**Figure 4** shows antibody blocking studies of TCGF (T cell growth factor) activity in supernatant from GP+envAM12 and GP+E86 cells transduced with pWZLIL2/B7M. 1µg/ml of irrelevant (UPC10) or neutralising anti-IL-2 (5334.2) antibodies were added to 1:4 dilutions of supernatants. ³H thymidine incorporation was measured and compared to the same dilutions without antibodies.
**Figure 5A** shows the proliferative response of CTLL cells to cytokines present in supernatant from AM12 cells transduced with pWZLIL4/B7M.
**Figure 5B** shows the proliferative response of CTLL cells to cytokines present in supernatant from AM12 cells transduced with pWZLIL4/IL2M measured in the absence or presence of neutralising IL-2 and/or IL-4 antibodies at 1µg/ml. Results are presented as cpm of incorporated ³H thymidine after a 4hr pulse.
**Figure 6** shows the Jurkat costimulation assay with GP+E86 and GP+envAm12 packaging cells, transduced with vector pWZLIL2B7M as stimulator cells. Bars indicate IL-2 production in response to PHA-P and GP+envAM12, or GP+E86 packaging cells as stimulator cells, in the presence of Jurkat cells, and in the absence or presence of antibodies (at 1 µg/ml) as indicated. Abbreviations: AM12=GP+envAM12.
**Figure 7** shows Table 1 which illustrates the cytokine production by the transduced cells quantified by ELISA or CTLL assay for measurement of biological activity.
**Figure 8** shows Table 2 which illustrates cell surface expression of B7 and IL2 as measured by flow cytometry on clones of NIH/3T3, 32Dp210, K562 and OIB cells.

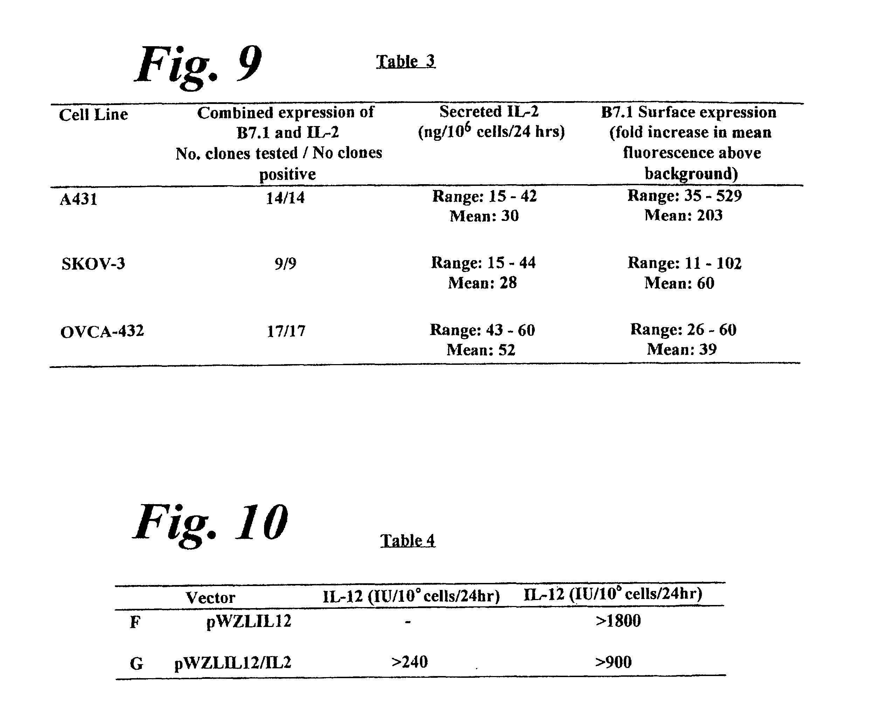

**Figure 9** shows Table 3 which illustrates expression of IL-2 and B7.1 in human tumour cells.
**Figure 10** shows Table 4 which illustrates the biological activity of cytokines (IL-2 and IL-12) in Fusagene vector transduced Cos 7 cells.

### Detailed description of the invention

The nucleic acid construct of the present invention may be provided as an isolate, in isolated and/or purified form. The nucleic acid may be wholly or partially synthetic and may include genomic DNA, cDNA or RNA.

The nucleic acid construct may comprise a number of sequences encoding polypeptides, each sequence being separated by a linker sequence encoding a tissue specific or ubiquitous endoprotease cleavage site. The construct may be readily prepared by the skilled person using the information and references herein and techniques known in the art (for example, see Sambrook, Fritsch and Maniatis, "molecular Cloning, A laboratory Manual, Cold Spring Harbor laboratory press, 1989, and Ausubel et al, Short Protocols in Molecular Biology, John Wiley and Sons 1992). These techniques include (i) the use of the polymerase chain reaction (PCR) to amplify samples of such nucleic acid, e.g. from genomic sources, (ii) chemical synthesis, or (iii) preparing cDNA sequences. Modifications to the nucleic acid sequence may be made, e.g. using site directed mutagenesis, to lead to the expression of modified polypeptides or to take account of codon preference in the target cell used to express the construct.

In order to obtain expression of the nucleic acid construct, the sequences can be incorporated in a vector having control sequences operably linked to the nucleic acids to control its expression. Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, virus, phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1992.

The two or more polypeptides can then be expressed in the target cell by transforming the vector into target cells by (transfection or infection). In such cells, in which the vector is functional, the fusion protein, being the expression product of the nucleic acid construct, is expressed, generating the two or more polypeptides by cleavage of the fusion protein within the cell.

The present invention allows the polypeptides to be processed within the cell such that their normal biological activity is either maintained or modified. Preferably, at least one of the two or more polypeptides will be able to be secreted from the cell in a biologically active form.

Prokaryotic and eukaryotic cells may be used for this purpose as known in the art, for example, strains of E. coli, yeast, and eukaryotic cells such as COS or CHO cells. However, in accordance with the present invention the target cell may be one which lacks expression of certain polypeptides or where the expression of further polypeptides may effect other biological processes. Further, the target cells may be chosen so that one of the two or more polypeptides can be secreted from the cells into the surrounding tissue. This will have many molecular implications, for example immune gene therapy of cancer. Although the present invention may be utilised on any cell type, suggested cell lines include fibroblasts, epithelial, muscular, neuronal, parenchymal, endocrine and bone marrow derived cells of various type and origin.

Depending on the target cell, the linker sequence encoding the endoprotease cleavage site may have to be modified to guarantee that the cleavage site is recognised by an endoprotease common to that target cell type. This is within the capabilities of the skilled person using the information and references contained herein and common general knowledge. It is preferred that the endoprotease is furin which is expressed in a wide variety of tissues and is highly conserved among eukaryotic species. However, the use of tissue specific endoprotease cleavage sites, rather than furin will allow a further level of specificity, resulting in production of the desired biologically active components in specific tissues. This introduces a further level of targeting (in addition to tissue specific delivery and transcription) and provides an additional method for delivery of cytotoxic compounds and/or drug convertase genes.

The introduction of the nucleic acid construct in a target cell may take place in vivo or ex vivo by way of gene therapy. This is discussed below.

The polypeptides encoded by the nucleic acid construct may be active portions, fragments, or derivatives of polypeptides or genes. An "active portion" of a polypeptide or gene is a peptide which is less than said full length wild-type polypeptide, but which retains its essential biological activity. The polypeptides will be chosen depending on their function and the particular target cell. For example, polypeptides may be chosen that are not normally expressed in the target cell or polypeptides that can be secreted from the target cell into the surrounding tissue. For example, the construct may encode for cytotoxic compounds or drug convertases. The essential biological activity required by the polypeptides may be determined by routine testing of the polypeptides expressed by, for example, the interaction of other binding proteins e.g. antibodies.

Other methods for determining biological activity are well known to the person skilled in the art. Examples of such methods include: conversion of substrate to product (e.g. enzymes); generation of a product (e.g. hormones) and measurement of the product either directly or through its biological activity (e.g. stimulated growth of responsive cells); binding or other interactions with proteins or other targets such as DNA or RNA sequences (e.g. DNA binding proteins); altered level of production of specific RNA or proteins (transcription factors); colorimetrically measured changes brought about by reduced levels of product (enzymes); staining for the protein itself, its binding ligand or other products of its activity.

The nucleic acid construct of the present invention encoding the two or more authentic biologically active polypeptide could be used in a method of gene therapy, to treat a patient who is unable to synthesize the active polypeptides or unable to synthesize such polypeptides in specific cells or tissues at all or at the required amount or in the appropriate molar ratios, thereby providing the effects manifested by such wild-type, modified or novel polypeptides; or enabling the target cells to produce and/or secrete the polypeptides into the surrounding medium.

Vectors such as viral vectors have been used in the prior art to introduce genes into a wide variety of different target cells. Typically the vectors are exposed to the target cells so that transfection can take place in a sufficient proportion of the cells to provide a useful therapeutic or prophylactic effect from the expression of the desired polypeptide. The transfected nucleic acid may be retained in the cell transiently or permanently either as an episome, as an autonomously replicating polynucleotide, or by being incorporated into the nuclear or mitochondrial genome of the cells, for example tumour cells, thus providing either transient or long lasting effects. In the case of transient expression or transient effects the treatment may be repeated periodically.

A variety of vectors, both viral vectors and plasmid vectors, are known in the art, see US Patent No. 5,252,479 and WO 93/07282. In particular, a number of viruses have been used as gene transfer vectors, including papovaviruses, such as SV40, vaccinia virus, herpes viruses, including HSV and EBV, and retroviruses. Many gene therapy protocols in the prior art have used disabled murine retroviruses.

As an alternative to the use of viral vectors, other known methods of introducing nucleic acid into cells includes electroporation, calcium phosphate co-precipitation, mechanical techniques such as microinjection, transfer mediated by liposomes, by high velocity projectiles and direct DNA uptake and receptor-mediated DNA transfer, or even by protoplast or cell fusion.

As mentioned above, the aim of gene therapy using nucleic acid encoding the two or more polypeptides, or an active portions thereof or inactive pre- or pre-pro-proteins, is to increase the amount of the expression product of the nucleic acid in cells in which the level of the wild-type polypeptides is absent or present only at reduced levels. Alternatively, the aim may be to modify target cells so that they produce particular polypeptides not usually produced in that cell. Such treatment may be therapeutic in the treatment of cells which are, for example, cancerous or prophylactic in the treatment of individuals known to be deficient in particular polypeptides. The treatment may also consist of prophylatic or therapeutic vaccination (e.g. by tumour cell vaccines thus modified), or for expression of either active drug convertase or inactive drug convertases which are activated after such proteolytic processing.

The present invention therefore also provides constructs as discussed above for use in medical treatment and use of the constructs, in the preparation of a medicament for the treatment of cancer cells.

### MATERIALS AND METHODS

### Cloning of the fusion vectors

Two hIL2/hB7.1 fusion vectors were constructed, one containing a full-length hIL-2 and a full-length hB7.1 cDNA, and a second vector containing full-length hIL-2 and a cDNA encoding only the mature hB7.1 product. In both vectors the IL-2 and B7.1 coding sequences were separated by a furin cleavage site, flanked at each end by two additional glycine residues to make the cleavage site spatially more accessible. This sequence (GGC-GGC-AGG-GGT-CGA-CGC-GGC-GGC) contained a *Sal*I recognition site (indicated in bold), and encodes Gly.Gly.Arg.Gly.Arg.Arg.Gly.Gly. Using appropriate PCR primers the IL-2 and B7.1 cDNA sequences were amplified in a number of fragments which were individually sequenced to confirm the absence of mutations. These fragments were ligated to generate retroviral vectors containing the Moloney Murine Leukaemia Virus (MoMLV) long terminal repeat (LTR) sequences and the full length hIL-2 (nucleotides 48 to 507) fused via the furin site to either a full-length hB7.1 (nucleotides 318 to 1184) or the mature B7.1 (nucleotides 396 to 1184) as depicted in Figure 2, using a pUC derived plasmid backbone. Similar strategies apparent to those skilled in the art, were employed for construction of other vectors depicted in figure 2C to figure 2G.

### Production of the Fusagene retroviral vectors

The generated plasmids were transfected into the ecotropic retroviral packaging cell line (GP+E-86), the culture supernatant of which after selection in 4-8µg/ml blasticidin S was used to infect the amphotropic packaging cell line (GP+env-AM12) cells. The culture supernatant of individual clones of the GP+envAM12 cells were used to infect different target cells essentially as previously described (Gäken et al., 1997).

### Analysis of cytokine production and B7.1 expression

Cytokine production: The level of IL-2 and IL-4 was measured either by ELISA, using the antibodies JES6-1A12 and 11B11 for the capture and detection of IL-2, respectively; or JES6-5H4 and BVD6-24G2 (Pharmingen San Diego CA) for the capture and detection of IL-4. The biological assay for these cytokines was based on ³H-thymidine incorporation by CTLL cells. This assay was performed in the presence of 1µg/ml of either an irrelevant antibody (clarified ascites of clone UPC10, mouse IgG2a) or neutralising antibodies to either IL-2 (purified, clone 5334.2, mouse IgG2a, R&D Systems), or IL-4 (11B11).

hB7-1 expression: Expression of B7.1 was measured by flow cytometry, using either the counter-receptor CTLA4, or FITC conjugated mouse monoclonal antibody (clone BB1, IgM, PharMingen). The CTLA4 was isolated from tissue culture supernatant of CTLA4Ig-producing J558L cells, a gift from Dr Christian Döhring, Basel Institute for Immunology, Basel, Switzerland (Döhring et al., 1994), and was used in conjunction with a FITC-conjugated goat anti-human IgG (Sigma). The biological activity of the hB7.1 was confirmed by a CD28 mediated T-cell co-stimulation assay. The hB7.1 expressing cells were co-cultured in the presence or absence of a monoclonal anti-CD25 (clone 33B.1 rat IgG2a, Immunotech) with the Jurkat T-cells which had received T-cell receptor/CD3 complex activation in the presence of 2-4 µg/ml PHA-P, followed by the assessment of IL-2 production by ELISA, as described above.

### RESULTS

### Vector construction

Human IL-2/B7.1. Two retroviral vectors were constructed in which the coding sequences for B7.1 and IL-2 were cloned together with a furin recognition site between the 2 cDNA's (Fig 2). The vectors contain the Moloney murine leukaemia virus (MoMLV) long terminal repeats (LTR) which controls the transcription of a single polycistronic messenger RNA. In both vectors the IL-2 coding sequence is followed by either the coding sequence for mature B7.1 lacking the signal peptide (pWZLIL2/B7M, see Fig. 2a), or the full coding sequence of B7.1, containing the signal peptide (pWZLIL2/B7F, see Fig. 2b). The IL-2/B7.1 sequence is followed by the encephalomyocarditis virus (ECMV) internal ribosome entry site (IRES), which allows the translation of the blast gene conferring resistance to blasticidin S. In both vectors the IL-2 signal peptide is responsible for the transport of the chimeric IL-2/B7.1 protein into the endoplasmic reticulum (ER). The chimeric protein encoded by these vectors should be transported to the golgi with the B7.1 transmembrane domain anchoring the protein to the membrane. This will allow the cleavage of the chimeric protein by furin in the golgi apparatus, resulting in generation of a cytoplasmic membrane bound B7.1 and secreted IL-2.

In a control vector, pWZLB7/IL2M (Fig. 2c), the relative position of the IL-2 and B7.1 cDNA were reversed. In this vector the signal peptide of B7.1, instead of IL-2, is used for transport into the ER. In this configuration the presence of the B7.1 transmembrane region prevents entry of the c-terminal portion of the fusion protein (containing the B7.1 cytoplasmic region, furin cleavage site and mature IL-2) into the lumen of the ER. Therefore, cleavage of the furin site cannot take place because the furin recognition site remains outside the golgi.

### Vectors encoding IL-4/IL-2, and IL-4/B7.1.

To investigate the general applicability of this approach the same strategy was used to construct other fusion proteins. The vector pWZLIL4/B7M encodes a chimeric IL-4/B7.1 protein containing the furin site between B7.1 and IL-4 (Fig. 2d). The vector pWZLIL4/IL2M encodes a chimeric IL-4/IL-2 again separated by a furin cleavage site (Fig. 2e). These vectors should encode secreted cytokines and a membrane anchored B7.1.

Each of the plasmids described was introduced into retroviral packaging cells and following selection for blast resistance both amphotropic and ecotropic retrovirus producer cell lines were isolated. These vectors were used for the transduction of target cells and analysis of expression and functional activity of the encoded fusion genes.

### Vectors encoding IL-12 (p40 and p35), and IL-2/IL-12

Two further vectors were constructed (see Fig. 2 F and G). vector F contains the IL-12 p40 and p35 cDNA with a furin recognition site between the cDNAs. Vector G is a triple vector with two IL-12 cDNAs (p35 and p40) and the IL-2 coding sequence, having two furin sites separating the three coding sequences.

In both vectors F and G the only signal peptide is present in the p40 fragment. The signal peptide for p35 and IL-2 were removed to overcome probable interference due to the presence of multiple signal peptides in the chimeric fusion protein.

### The presence of furin recognition sequence enables the cleavage of the chimeric protein into its constituent products.

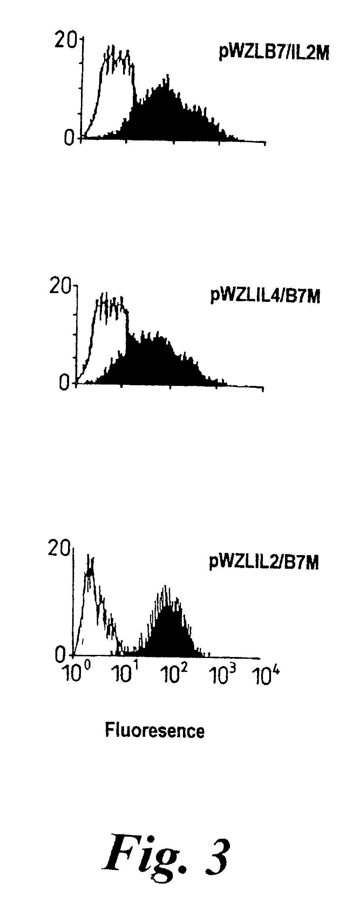

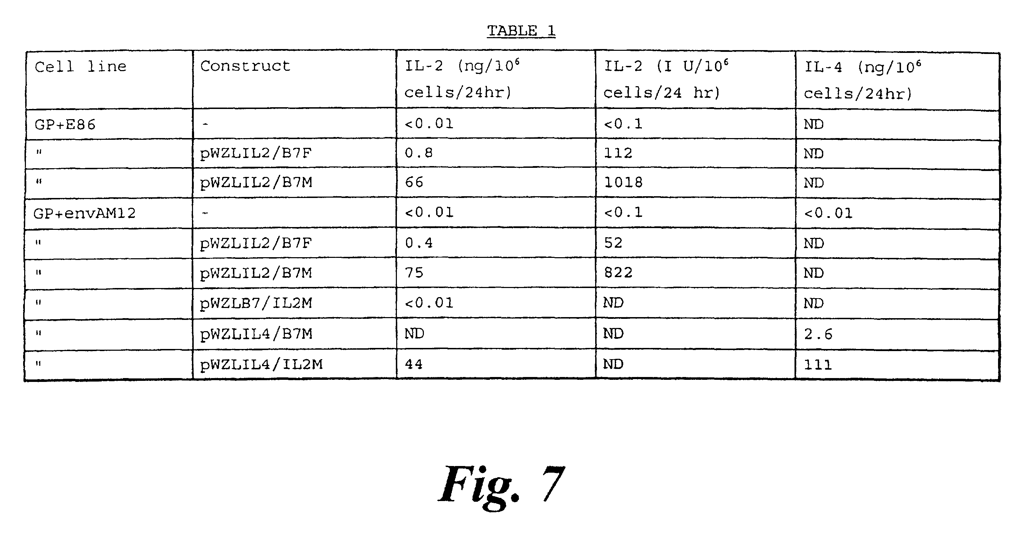

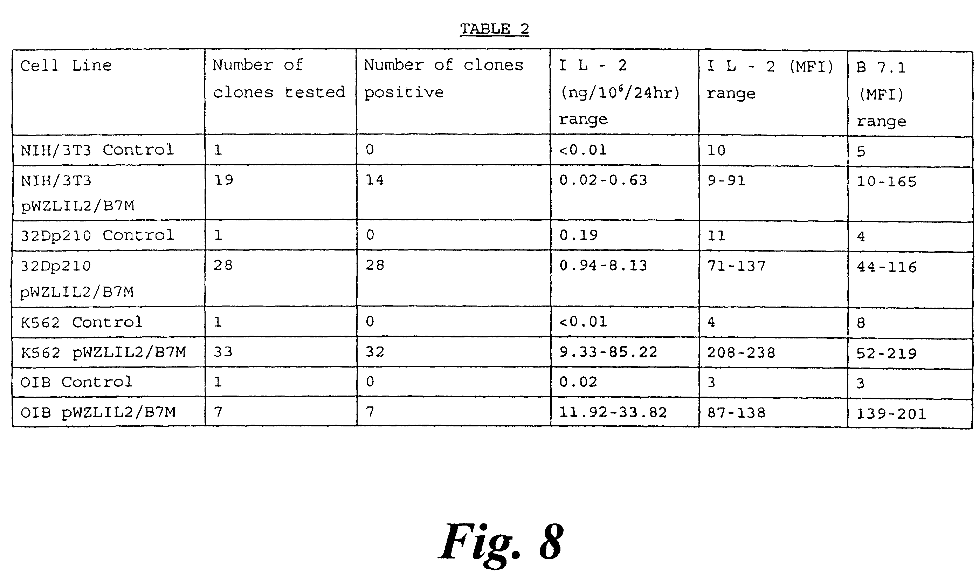

Cells expressing constructs that contain the furin recognition sequence show clear evidence of cytokine production as measured by ELISA (Fig. 7. Table 1). When the furin target sequence is present between IL-4 and IL-2 both cytokines are secreted into the culture medium. Similarly, in vectors encoding the chimeric IL-2/B7.1 or IL-4/B7.1 with an intervening furin site there is cell surface expression of B7.1 as measured by FACS analysis (Fig. 3). However, a small fraction of the chimeric protein remains uncleaved. This is demonstrated by the cell surface detection of the cytokines (Fig. 8 Table 2), and ELISA based detection of the chimeric IL-2/IL-4 in assays which used IL-2 antibodies for capture and IL-4 antibodies for detection, or vice versa (data not shown). The latter experiments demonstrated that the level of uncleaved product was dependent on cell type, probably reflecting their endogenous level of furin expression. Interestingly, if both the B7.1 moiety and the IL-2 contained signal peptides there was substantially (more than 80 fold) less cytokine released (Table 1). The precise mechanism for this reduced production of cytokine is not clear but is likely to be due to interference with either insertion of the fusion protein into the ER, or its subsequent furin cleavage in the golgi. The generation of a membrane anchored B7.1 and secreted cytokine from cells infected with these vectors is dependent on the presence of the furin cleavage site. In the absence of the furin site there is no cytokine secretion into the culture medium (data not shown). Similarly, if the furin cleavage site is placed in a configuration preventing its entry into the lumen of the ER, thus preventing its access to furin in the golgi, there is no cytokine release. This is clearly demonstrated in cells transduced with pWZLB7/IL2M (see figure 2C) in which the furin recognition site is linked to the cytoplasmic domain of the B7.1 molecule (Table 1). In this configuration the furin cleavage site remains in the cytoplasm, does not enter the lumen of ER, and gains no access to furin in the golgi apparatus. However, there is clear evidence of B7 surface expression as determined by FACS analysis (Fig 3).

### Biological activity of the fusion gene products

### CTLL proliferation assays on supernatants of cell lines infected with pWZLIL2/B7M and pWZLIL2/B7F.

To determine the biological activity of the secreted cytokines their effect on proliferation (³H-thymidine incorporation) of the cytokine dependent CTLL cells was measured. Cells transduced with the vector pWZLB7/IL2M produced significantly more T cell growth factor (TCGF) than those transduced with pWZLB7/IL2F. This is consistent with the ELISA based measurements of the cytokine levels (See Table 1).

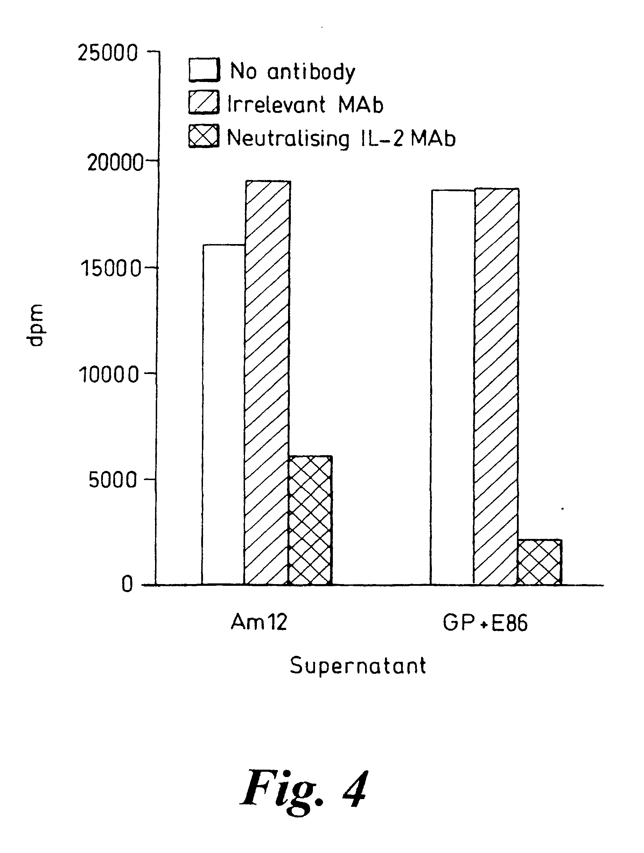

In order to confirm that IL-2 in the packaging cell supernatants induced proliferation in CTLL cells, antibody blocking studies were performed. CTLL proliferation in response to supernatants could be blocked by a neutralising IL-2 antibody, but much less efficiently by the irrelevant antibody (Fig. 4).

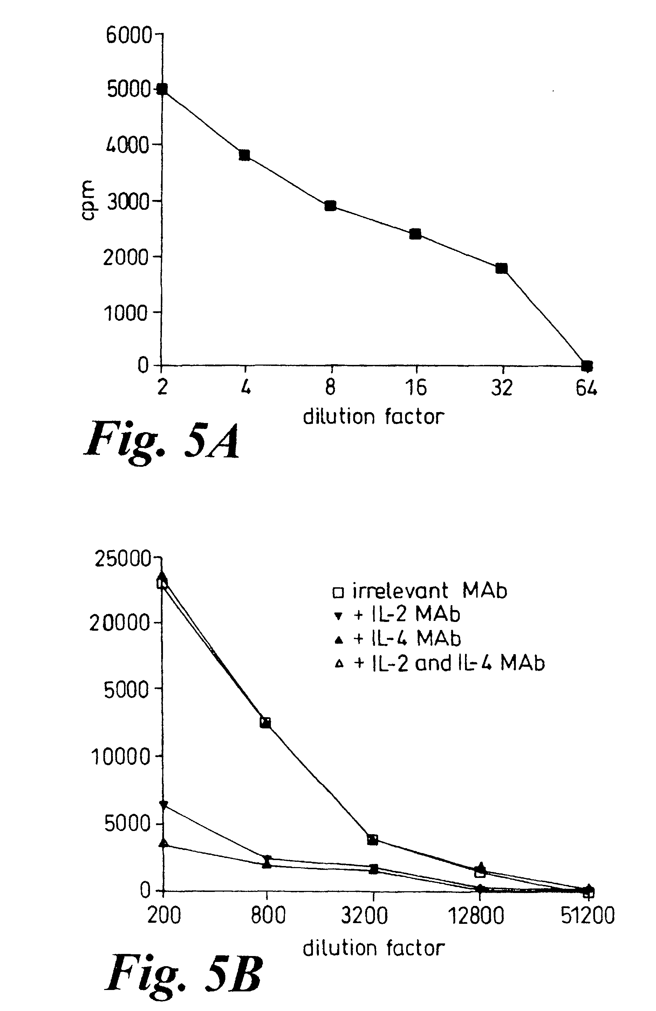

Similarly the bioactivity of IL-2 and IL-4 produced by cells transduced with either pWZLIL4/B7M or pWZLIL4/IL2M was determined on CTLL cells (Fig 5). These cells are much more responsive to IL-2 than IL-4 therefore the addition of neutralising IL-4 antibody has little effect on the proliferation of the CTLL cells. However in the presence of the neutralising IL-2 antibody the addition of IL-4 antibody decreases the thymidine incorporation indicating the bioactivity of both IL-2 and IL-4 produced by these cells.

### Jurkat costimulation assay, using as stimulator cells, GP+E86 and GP+envAm12 packaging cells, transduced with vector pWZLIL2/B7M

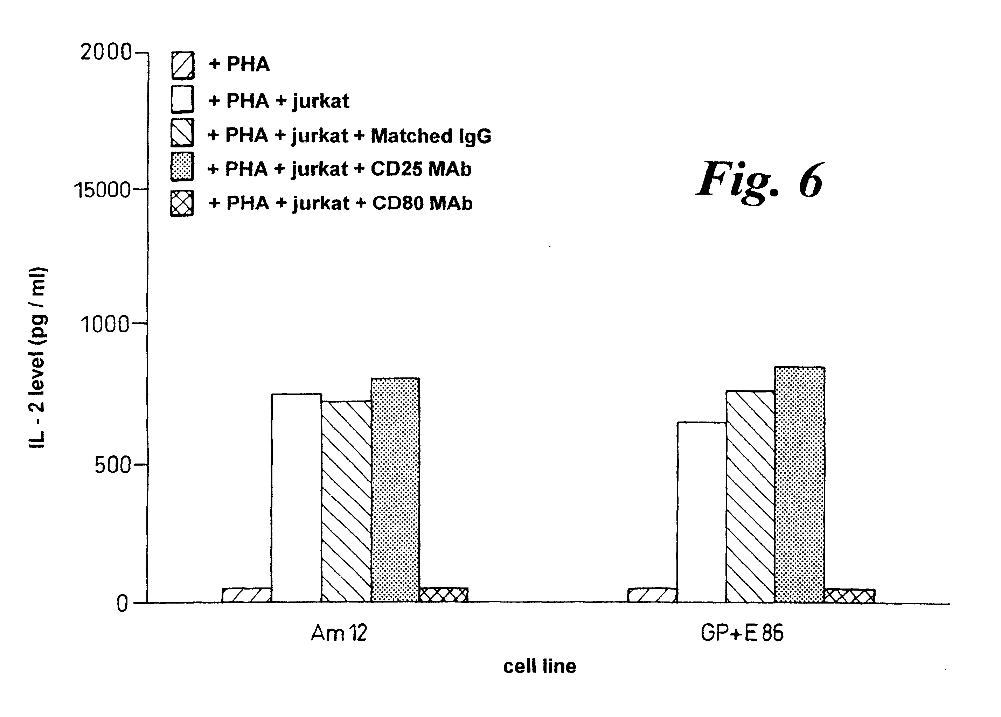

To determine that the B7.1 molecules expressed on the surface of transduced cells were biologically active a Jurkat costimulation assay was performed. The co-culture of Jurkat cells with the pWZLIL2/B7M transduce cells and PHA-P resulted in increased levels of IL-2 in the culture supernatant (Fig 6). This activation of Jurkat cells, as measured by IL-2 production, was B7.1 dependent and could not be blocked by inhibition of the IL-2 receptor (CD25). Therefore, the pWZLIL2/B7M transduced cells expressed biologically active B7.1 molecules able to co-stimulate the activation of PHA-P treated Jurkat cells.

### IL-12 biological activity: T-cell activation by supernatant from Cos 7 cells transduced with the IL-12 containing vectors

The biological activity of IL-12 in cells transduced with vector F (see Fig. 2 F), and IL-12 plus IL-2 in cells transduced with vector G were confirmed as described below.

The biological activity of IL-12 is determined by induction of growth in activated human T cells. This growth is measured by the incorporation of radioactive thymidine into the cellular DNA. An assay was performed according to Coligan et al., in Current Protocols in Immunology, Chapter 6.16 Lymphoblast Proliferation Assay for IL-12. The biological activity of Il-2 was measured with a standard CTLL assay as described above. Table 4 shows the level of biologically active IL-12 and IL-2 secreted into the culture medium of cells transduced with vectors F and G.

### Transduction of target cells

To investigate the expression of both B7.1 and IL-2 in different cells a panel of tissue culture cells were infected and the expression of B7.1 and IL-2 was measured. The following cell lines were infected with the pWZLIL2/B7M fusion vector and assessed for the cell surface expression of B7.1 and the secretion of IL-2 into the culture medium; NIH/3T3 mouse fibroblasts, 32Dp210 murine myeloid cells, K562 human erythroid leukaemia and OIB a human ovarian adenocarcinoma. To determine the presence of uncut fusion products the cells were also tested by FACS analysis for the presence of IL-2 on the cell surface (see Table 2). The Il-2-B7.1 vector has also been used to infect 3 further cell lines, namely, A431 human endometrial tumour cell line and two human ovarian cell lines SKOV-3 and OVCA-432. A total of 40 clones were tested and all were positive for B7.1 surface expression and IL-2 secretion into the supernatant (see Table 3).

Cos 7 cells were transduced by electroporation (see Sambrook, Fritsch and Maniatis, "Molecular Cloning, a Laboratory Manual", Cold Spring Harbour Laboratory Press, 1989).

### DISCUSSION

The vector system described here utilises the presence of a furin recognition site to express both secreted and membrane bound, biologically active, proteins from a single cistron. As demonstrated such vectors may be plasmids, or either RNA or DNA virus based vectors.
The use of endoprotease cleavage sites enables the expression of a monocistronic transcript that is translated into a chimeric fusion product which is then processed by endoprotease cleavage to produce the constituent components. Therefore, the molar ratio of the protein products will be determined by the frequency of their representation in the vector. If the vector contains one IL-2 and one IL-4 coding sequence, the two cytokines will be expressed in equimolar ratio; if there is two IL-2 and one IL-4 coding sequences, the two cytokines will be expressed in the corresponding 2:1 molar ratio. Furthermore, the fact that seven constructs containing different combinations of genes all resulted in production of biologically active proteins indicates that this approach has a broad applicability.

The variation in the level of IL-2 produced in different cell lines is likely to be a reflection of the variation in furin levels and/or efficiencies of different processes involved in gene expression, and/or stability of the products. The level of IL-2 production by NIH/3T3 cells is much lower than that observed in 32Dp210, K562 and OIB cells. This is corroborated by the higher level of FACS detectable IL-2 on the surface of these NIH/3T3 cell. The presence of IL-2 on the cell surface is not due to non-specific binding of IL-2 to the cells because when IL-2 producing cells were mixed with non-producing cells two discreet populations were detectable by FACS analysis (results not shown) . Therefore, the IL-2 on the cell surface is probably still fused to B7 indicating the inefficient processing of the chimeric protein by the endogenous level of furin in these cells. This reduced level, or inefficiency of, furin mediated processing has also been observed in COS cells. Only a small fraction of pro von Willebrand factor (Wise et al., 1990 Proc. Natl. Acad. Sci. U.S.A. 87, 9378-9382) and rat proinsulin I (Smeekens et al., 1992 Proc. Natl. Acad. Sci. U.S.A. 89, 8822-8826), both of which are normally processed by furin into their biologically active products, were produced in these cells. Co-transfection of COS cells with vectors encoding furin resulted in increased secretion of protein in both these systems (Wise et al., 1990 Proc. Natl. Acad. Sci. U.S.A. 87, 9378-9382; Smeekens et al., 1992 Proc. Natl. Acad. Sci. U.S.A. 89, 8822-8826). Therefore, in cells with low endogenous levels of furin introduction of furin expression vectors might increase the furin levels, thus resulting in increased cleavage of chimeric proteins incorporating a furin cleavage site. Furthermore, changes to the furin cleavage site itself could improve the efficiency. In chinese hamster ovary cells the amino acid sequence Arg-X-Lys-Arg enables 50% cleavage of the precursor protein, whilst Arg-X-Arg-Arg results in only 30% cleavage (Watanabe et al., 1992 J. Biol. Chem. 267, 8270-8274). Finally, the use of tissue specific endoprotease cleavage sites, rather than the ubiquotously expressed furin, will introduce a further level of specificity, resulting in production of the desired biologically active components in specific tissues. This introduces a further level of targeting (in addition to tissue specific delivery and transcription) and is likely to be a particularly attractive strategy in a number of applications including gene therapy of tissue or organ specific genetic disorders be they inherited or otherwise, or for the delivery of cytotoxic, cytostatic or differentiation promoting proteins or polypeptides, or proteins with catalytic activities enabling them to convert pro-drugs to active drugs (drug convertases) in a cell type or tissue specific manner. The use of such tissue specific endoprotease recognition sites in order to achieve tissue specific processing and activation of otherwise inactive chimeric proteins is another advantage of the present invention.

In addition to the demonstrated activity of the fusion strategy in plasmid and retrovirus based vectors, already described, the present inventors have also obtained evidence of activity in adenovirus based vectors. The B7.1/IL-2 cassette has been cloned into an adenoviral vector (the E1 deleted Ad 5 vector). This vector was used to infect an established cell line (HeLa) and primary acute myeloid leukaemia (AML) cells isolated from three AML patients. Analysis of each of the cell populations have demonstrated high level surface expression of B7.1 and production of secreted IL-2 in the culture supernatant of the infected cells in excess of 20ng/10⁶ cells/24hr.

## Claims

1. A nucleic acid construct for expressing two or more individual biologically active polypeptides in a cell comprising a first sequence encoding a first polypeptide and a second sequence encoding a second polypeptide, said first and second sequences being separated by a linker sequence encoding an endoprotease cleavage site.

2. A nucleic acid construct according to claim 1 further comprising spacer sequence positioned either side of the linker sequence encoding the endoprotease cleavage site.

3. A nucleic acid construct according to claim 2 wherein the spacer sequence comprises nucleic acid encoding at least 2 amino acids.

4. A nucleic acid construct according to any one of the preceding claims wherein the linker sequence comprises a nucleic acid sequence encoding Arg.X.Y.Arg where X is any amino acid residue and Y is Lysine or Arginine.

5. A nucleic acid construct according to claim 4 wherein the linker sequence comprises nucleic acid sequence which encodes Arg.Gly.Arg.Arg., Arg.Pro.Lys.Arg., or Arg.Tyr.Arg.Arg.

6. A nucleic acid construct according to any one of the preceding claims wherein the linker sequence comprises AGG-GGT-CGA-CGC, CGG-CCT-AAG-AGG, or AGG-TAC-CGC-AGG.

7. A nucleic acid construct according to any one of claims 2 to 6 wherein the linker sequence and the spacer sequence encode Gly.Gly.Arg.Gly.Arg.Arg.Gly.Gly., Gly.Arg.Pro.Lys.Arg.Pro., or Gly.Gly.Arg.Tyr.Arg.Arg.Gly.Gly.

8. A vector comprising a nucleic acid construct according to any one of the preceding claims.

9. A host cell comprising a vector according to claim 8 or a nucleic acid construct according to any one of claims 1 to 7.

10. An ex vivo or in vitro method of expressing two or more polypeptides in a cell comprising the steps of
a) constructing a nucleic acid construct according to any one of claims 1 to 7; and
b) transfecting said cell with said nucleic acid construct such that said nucleic acid construct is expressed to produce a fusion protein comprising a first and second polypeptides linked by an endoprotease cleavage site capable of being cleaved by an endoprotease within the cell to generate individual biologically active first and second polypeptides.

11. An ex vivo or in vitro method according to claim 10 wherein the nucleic acid construct is art of nucleic acid vector.

12. An ex vivo or in vitro method according to claim 11 wherein the vector is a viral vector.

13. An ex vivo or in vitro method according to claim 12 wherein the viral vector is a retroviral vector or an adenovirus based vector.

14. An ex vivo or in vitro method according to claim 12 or 13 wherein the viral vector is firstly transfected into a viral packaging cell line to produce virus which is then used to transfect or infect the cell.

15. A method for producing two or more polypeptides comprising the steps of
a) constructing a nucleic acid construct according to any one of claims 1 to 7;
b) transfecting a cell with said nucleic acid construct such that said nucleic acid construct is expressed to produce a fusion protein comprising a first and second polypeptides linked by an endoprotease cleavage site capable of being cleaved by an endoprotease within the cell to generate individual biologically active first and second polypeptides; and
c) isolating said two or more polypeptides from the cell individually or in combination.

16. A method according to claim 15 wherein the first and second polypeptide are subunits of a protein.

17. A method according to claim 16 wherein the first and second polypeptides are subunits of IL-12

18. A method according to claim 17 further comprising one or more additional steps of preparing the polypeptides for industrial, diagnostic or therapeutic purposes.

19. An ex vivo or in vitro method of producing a cellular vaccine comprising the steps of
a) constructing a nucleic acid construct according to any one of claims 1 to 7 wherein the first and/or second polypeptides are capable of invoking an immune response;
b) inserting said nucleic acid construct into a nucleic acid vector;
c) transforming a cell with said vector such that the first and/or second polypeptides are either secreted or expressed and displayed on the surface of the cell.

20. An ex vivo or in vitro method according to claim 19 wherein the cell is a tumour cell.

21. An ex vivo or in vitro method according to claim 19 or claim 20 wherein the first and second polypeptides are subunits of IL-12.

22. A nucleic acid construct according to any one of claims 1 to 7 or a vector according to claim 8 for use in a method of treatment of the human or animal body.

23. Use of a nucleic acid construct according to claims 1 to 7 or a vector according to claim 8 in the preparation of a medicament for use in a method of gene therapy.

## Revendications

1. Construction d'acide nucléique pour l'expression de deux ou plusieurs polypeptides individuels biologiquement actifs dans une cellule, comprenant une première séquence codant pour un premier polypeptide et une seconde séquence codant pour un second polypeptide, lesdites première et seconde séquences étant séparées par une séquence d'un linker codant pour un site de scission d'endoprotéase.

2. Construction d'acide nucléique selon la revendication 1 comprenant de plus une séquence d'espacement placée de chaque côté de la séquence du linker codant pour le site de scission d'endoprotéase.

3. Construction d'acide nucléique selon la revendication 2 où la séquence d'espacement comprend un acide nucléique codant pour au moins deux acides aminés.

4. Construction d'acide nucléique selon l'une quelconque des revendications précédentes où la séquence du linker comprend une séquence d'acide nucléique codant pour Arg. X. Y. Arg. où X est tout résidu d'acide aminé et Y est Lysine ou Arginine.

5. Construction d'acide nucléique selon la revendication 4 où la séquence du linker comprend une séquence d'acide nucléique qui code pour Arg. Gly. Arg. Arg., Arg. Pro. Lys. Arg., ou Arg. Tyr. Arg. Arg.

6. Construction d'acide nucléique selon l'une quelconque des revendications précédentes où la séquence du linker comprend AGG-GGT-CGA-CGC, CGG-CCT-AAG-AGG, ou AGG-TAC-CGC-AGG.

7. Construction d'acide nucléique selon l'une quelconque des revendications 2 à 6 où la séquence du linker et la séquence d'espacement code pour : Gly. Gly. Arg. Gly. Arg. Arg. Gly. Gly., Gly. Arg. Pro. Lys. Arg. Pro., ou Gly. Gly. Arg. Tyr. Arg. Arg. Gly. Gly.

8. Vecteur comprenant une construction d'acide nucléique selon l'une quelconque des revendications précédentes.

9. Cellule hôte comprenant un vecteur selon la revendication 8 ou une construction d'acide nucléique selon l'une quelconque des revendications 1 à 7.

10. Méthode ex vivo ou in vitro d'expression de deux polypeptides ou plus dans une cellule, comprenant les étapes de
a) construire une construction d'acide nucléique selon l'une quelconque des revendications 1 à 7 ; et
b) transfecter ladite cellule avec ladite construction d'acide nucléique de façon que ladite construction d'acide nucléique soit exprimée pour produire une protéine de fusion comprenant un premier et un second polypeptides enchaînés par un site de scission d'endoprotéase pouvant être scindé par une endoprotéase dans la cellule pour générer des premier et second polypeptides individuels biologiquement actifs.

11. Méthode ex vivo ou in vitro selon la revendication 10 où la construction d'acide nucléique est du type vecteur d'acide nucléique.

12. Méthode ex vivo ou in vitro selon la revendication 11 où le vecteur est un vecteur viral.

13. Méthode ex vivo ou in vitro selon la revendication 12 où le vecteur viral est un vecteur rétroviral ou un vecteur à base d'adénovirus.

14. Méthode ex vivo ou in vitro selon la revendication 12 ou 13, où le vecteur viral est d'abord transfecté dans une lignée de cellules d'encapsidation virale pour produire un virus qui est alors utilisé pour transfecter ou infecter la cellule.

15. Méthode de production de deux polypeptides ou plus comprenant les étapes de :
a) construire une construction d'acide nucléique selon l'une quelconque des revendications 1 à 7 ;
b) transfecter une cellule avec ladite construction d'acide nucléique de façon que ladite construction d'acide nucléique soit exprimée pour produire une protéine de fusion comprenant un premier et un second polypeptides enchaînés par un site de scission d'endoprotéase pouvant être scindé par une endoprotéase dans la cellule pour générer des premier et second polypeptides individuels biologiquement actifs ; et
c) isoler lesdits deux polypeptides ou plus de la cellule, individuellement ou en combinaison.

16. Méthode selon la revendication 15 où les premier et second polypeptides sont des sous-unités d'une protéine.

17. Méthode selon la revendication 16 où les premier et seconde polypeptides sont des sous-unités de IL-12.

18. Méthode selon la revendication 17 comprenant de plus une ou plusieurs étapes additionnelles de préparation des polypeptides dans des buts industriels, de diagnostic ou thérapeutiques.

19. Méthode ex vivo ou in vitro de production d'un vaccin cellulaire comprenant les étapes de :
a) construire une construction d'acide nucléique selon l'une quelconque des revendications 1 à 7 où les premier et/ou second polypeptides sont capables d'invoquer une réponse immune ;
b) insérer ladite construction d'acide nucléique dans un vecteur d'acide nucléique ;
c) transformer une cellule avec ledit vecteur de façon que les premier et/ou second polypeptides soient sécrétés ou exprimés et présentés à la surface de la cellule.

20. Méthode ex vivo ou in vitro selon la revendication 19 où la cellule est une cellule de tumeur.

21. Méthode ex vivo ou in vitro selon la revendication 19 ou la revendication 20 où les premier et second polypeptides sont des sous-unités de IL-12.

22. Construction d'acide nucléique selon l'une quelconque des revendications 1 à 7 ou un vecteur selon la revendication 8 à utiliser dans une méthode de traitement du corps humain ou animal.

23. Utilisation d'une construction d'acide nucléique selon les revendications 1 à 7 ou d'un vecteur selon la revendication 8 dans la préparation d'un médicament pour une utilisation dans une méthode de thérapie génique.

## Patentansprüche

1. Nucleinsäurekonstrukt zum Exprimieren zweier oder mehrerer individueller, biologisch aktiver Polypeptide in einer Zelle, umfassend eine erste Sequenz, die für ein erstes Polypeptid kodiert, und eine zweite Sequenz, die für ein zweites Polypeptid kodiert, wobei die erste und die zweite Sequenz durch eine Linker-Sequenz getrennt sind, die für eine Endoprotease-Spaltungsstelle kodiert.

2. Nucleinsäurekonstrukt nach Anspruch 1, das weiters eine an einer Seite der für die Endoprotease-Spaltungsstelle kodierenden Linker-Sequenz angeordnete Spacer-Sequenz umfasst.

3. Nucleinsäurekonstrukt nach Anspruch 2, worin die Spacer-Sequenz Nucleinsäure umfasst, die für zumindest 2 Aminosäuren kodiert.

4. Nucleinsäurekonstrukt nach einem der vorangegangenen Ansprüche, worin die Linker-Sequenz eine für Arg.X.Y.Arg kodierende Nucleinsäuresequenz umfasst, worin X ein beliebiger Aminosäurerest ist und Y Lysin oder Arginin ist.

5. Nucleinsäurekonstrukt nach Anspruch 4, worin die Linker-Sequenz eine Nucleinsäuresequenz umfasst, die für Arg.Gly.Arg.Arg., Arg.Pro.Lys.Arg. oder Arg.Tyr.Arg.Arg. kodiert.

6. Nucleinsäurekonstrukt nach einem der vorangegangenen Ansprüche, worin die Linker-Sequenz AGG-GGT-CGA-CGC, CGG-CCT-AAG-AGG oder AGG-TAC-CGC-AGG umfasst.

7. Nucleinsäurekonstrukt nach einem der Ansprüche 2 bis 6, worin die Linker-Sequenz und die Spacer-Sequenz für
Gly.Gly.Arg.Gly.Arg.Arg.Gly.Gly.,
Gly.Arg.Pro.Lys.Arg.Pro. oder
Gly.Gly.Arg.Tyr.Arg.Arg.Gly.Gly. kodieren.

8. Vektor, der ein Nucleinsäurekonstrukt nach einem der vorangegangenen Ansprüche umfasst.

9. Wirtszelle, die einen Vektor nach Anspruch 8 oder ein Nucleinsäurekonstrukt nach einem der Ansprüche 1 bis 7 umfasst.

10. Ex-vivo- oder In-vitro-Verfahren zur Expression zweier oder mehrerer Polypeptide in einer Zelle, folgende Schritte umfassend:
a) das Konstruieren eines Nucleinsäurekonstrukts nach einem der Ansprüche 1 bis 7; und
b) das Transfizieren der Zelle mit dem Nucleinsäurekonstrukt, so dass das Nucleinsäurekonstrukt so exprimiert wird, dass ein Fusionsprotein produziert wird, das ein erstes und ein zweites Polypeptid umfasst, die durch eine Endoprotease-Spaltungsstelle verbunden sind, die durch eine Endoprotease innerhalb der Zelle spaltbar ist, um individuelle, biologisch aktive erste und zweite Polypeptide zu erzeugen.

11. Ex-vivo- oder In-vitro-Verfahren nach Anspruch 10, worin das Nucleinsäurekonstrukt Teil eines Nucleinsäurevektors ist.

12. Ex-vivo- oder In-vitro-Verfahren nach Anspruch 11, worin der Vektor ein viraler Vektor ist.

13. Ex-vivo- oder In-vitro-Verfahren nach Anspruch 12, worin der virale Vektor ein retroviraler Vektor oder ein Vektor auf Adenovirus-Basis ist.

14. Ex-vivo- oder In-vitro-Verfahren nach Anspruch 12 oder 13, worin der virale Vektor zunächst in eine virale Packungszelllinie transfiziert wird, um ein Virus zu erzeugen, das dann zum Transfizieren oder Infizieren der Zelle verwendet wird.

15. Verfahren zur Produktion zweier oder mehrerer Polypeptide, folgende Schritte umfassend:
a) das Konstruieren eines Nucleinsäurekonstrukts nach einem der Ansprüche 1 bis 7;
b) das Transfizieren einer Zelle mit dem Nucleinsäurekonstrukt, so dass das Nucleinsäurekonstrukt exprimiert wird, um ein Fusionsprotein zu produzieren, das ein erstes und ein zweites Polypeptid umfasst, die durch eine Endoprotease-Spaltungsstelle verbunden sind, die von einer Endoprotease innerhalb der Zelle spaltbar ist, um individuelle, biologisch aktive, erste und zweite Polypeptide zu erzeugen; und
c) das Isolieren der zwei oder mehreren Polypeptide aus der Zelle, einzeln oder in Kombination.

16. Verfahren nach Anspruch 15, worin das erste und das zweite Polypeptid Untereinheiten eines Proteins sind.

17. Verfahren nach Anspruch 16, worin das erste und das zweite Polypeptid Untereinheiten von IL-12 sind.

18. Verfahren nach Anspruch 17, das weiters einen oder mehrere zusätzliche Schritte des Präparierens der Polypeptide für industrielle, diagnostische oder therapeutische Zwecke umfasst.

19. Ex-vivo- oder In-vitro-Verfahren zur Herstellung einer Zell-Vakzine, folgende Schritte umfassend:
a) das Konstruieren eines Nucleinsäurekonstrukts nach einem der Ansprüche 1 bis 7, worin das erste und/oder das zweite Polypeptid fähig sind, eine Immunreaktion hervorzurufen;
b) das Insertieren des Nucleinsäurekonstrukts in einen Nucleinsäurevektor;
c) das Transformieren einer Zelle mit dem Vektor, so dass das erste und/oder das zweite Polypeptid entweder sekretiert oder exprimiert und auf der Oberfläche der Zelle freigelegt werden.

20. Ex-vivo- oder In-vitro-Verfahren nach Anspruch 19, worin die Zelle eine Tumorzelle ist.

21. Ex-vivo- oder In-vitro-Verfahren nach Anspruch 19 oder 20, worin das erste und das zweite Polypeptid Untereinheiten von IL-12 sind.

22. Nucleinsäurekonstrukt nach einem der Ansprüche 1 bis 7 oder Vektor nach Anspruch 8 zur Verwendung in einem Verfahren zur Behandlung des menschlichen oder eines Tierkörpers.

23. Verwendung eines Nucleinsäurekonstrukts nach einem der Ansprüche 1 bis 7 oder eines Vektors nach Anspruch 8 zur Herstellung eines Medikaments zur Verwendung in einem Gentherapieverfahren.
